# EUROPÄISCHE PATENTSCHRIFT

(11) **EP 1 539 266 B1**
(45) Veröffentlichungstag und Bekanntmachung des Hinweises auf die Patenterteilung: **09.04.2008**
(21) Anmeldenummer: 03750300.0
(22) Anmeldetag: 26.08.2003
(51) Int. Cl.: A61L 29/16, A61L 31/16

(54) **MEDIZINISCHE VORRICHTUNG ZUR ARZNEIMITTELABGABE**
MEDICAL DEVICE FOR DISPENSING MEDICAMENTS
DISPOSITIF MEDICAL DESTINE A LA DISTRIBUTION D'UN MEDICAMENT

(30) Priorität: 20.09.2002 DE 10244847
(43) Veröffentlichungstag der Anmeldung: 15.06.2005
(60) Teilanmeldung: 07017424.8
(73) Patentinhaber: Bayer Schering Pharma Aktiengesellschaft, 13353 Berlin (DE)
(72) Erfinder: SPECK, Ulrich, 13465 Berlin (DE); SCHELLER, Bruno, 66111 Saarbrücken (DE)
(74) Vertreter: Weiss, Wolfgang
(86) Internationale Anmeldenummer: PCT/DE2003/002871
(87) Internationale Veröffentlichungsnummer: WO 2004/028582

(56) Entgegenhaltungen:
- WO-A-00/44414
- WO-A-00/45744
- WO-A-92/11890
- WO-A-92/15282
- DE-C- 4 225 553
- US-A- 5 370 614
- US-A1- 2002 123 505

## Beschreibung

Die Erfindung betrifft einen Ballonkatheter zur Arzneimittelabgabe für die selektive Therapie bestimmter Gewebeabschnitte oder Organteile und ein Verfahren zur Herstellung derartiger arzneimittelbeschichteter Geräte.

Zahlreiche Erkrankungen betreffen nicht den gesamten Organismus gleichzeitig sondern sind auf bestimmte Gewebearten, häufig auch auf sehr begrenzte einzelne Gewebebezirke oder Organteile beschränkt. Beispiele dafür finden sich unter den Tumor-, Gelenk- und Gefäßerkrankungen.

Die Pharmakotherapie auch dieser Erkrankungen erfolgt im allgemeinen durch orale oder intravenöse Gabe von Arzneistoffen, die sich im ganzen Körper verteilen und in vielen Fällen gerade bei schweren Erkrankungen unerwünschte Wirkungen in gesunden Geweben und Organen verursachen, die die therapeutische Anwendung begrenzen. Eine selektive Therapie der erkrankten Gewebe wurde mittels spezifisch an erkranktes Gewebe bindende Arzneistoffe (z. B. Antikörper) unter Beibehaltung des Applikationsweges oder durch selektive Verabreichung, z.B. durch direkte Injektion in das erkrankte Gewebe oder durch Zufuhr über Katheter zu den das erkrankte Gewebe versorgenden Blutgefäßen, erreicht. Im Falle der selektiven Verabreichung entstehen durch die meist kurze Wirkdauer der Arzneistoffe und die invasiven Verabreichungswege Probleme, da sich eine beliebig wiederholte Gabe verbietet. Bei selektiver Verabreichung über den das erkrankte Gewebe versorgenden Blutstrom ergibt sich das zusätzliche Problem ungenügen-der Extraktion der Arzneistoffe bei der raschen Passage des Blutes oder der Wirkstofflösung durch die Blutgefäße.

Diesen Problemen wurde bisher durch unterschiedliche pharmazeutische Präparate mit verzögerter Wirkstofffreigabe, arzneimittelfreisetzende Implantate oder für längere Zeit funktionsfähige selektive Zugangswege wie implantierte Katheter etc. begegnet.

Es ist bereits bekannt, die Oberfläche von in den Körper eingebrachten medizinischen Geräten, insbesondere Kathetern, mit Mitteln zur Verbesserung der Gleitfähigkeit oder zur Verhinderung der Blutgerinnung, jedoch ohne therapeutische Wirkung, zu beschichten.

Darüber hinaus werden Katheter mit speziellen Vorrichtungen versehen, um Arzneimittel in die Arterienwand zu injizieren, beispielsweise mittels Nadeln oder hohem Injektionsdruck über eine an der Gefäßwand anliegende Perforation der Katheterwand.

Andere Prinzipien beruhen darauf, die Kontaktzeit zwischen Arterienwand und einer über den Katheter applizierten Wirkstoffzubereitung zu verlängern, indem entweder der Blutstrom für einen entsprechenden Zeitraum unterbunden wird, z. B. Doppelballonkatheter mit zwischen den Ballons befindlicher mit der Arzneistofflösung gefüllter Kammer oder Hohlräumen zwischen der z.B. mit Wülsten versehenen Ballonaußenwand, wobei der Blutstrom durch einen den Ballon passierenden Kanal in begrenztem Maße aufrechterhalten werden kann.

Gemäß der US 5 102 402 werden Arzneistoffe in Form von Mikrokapseln zur verzögerten Wirkstofffreigabe lose in vorgeformten Vertiefungen von Ballonkathetern untergebracht. Nach Expansion des Ballons sollen die Mikrokapseln in die Gefäßwand gedrückt werden, dort verbleiben und den oder die Wirkstoffe langsam freisetzen. Zahlreiche Autoren schlagen auch vor, Arzneistoffe in Hydrogel eingebettet auf Ballonkatheter aufzubringen, wobei die Funktion des Hydrogels als Haftmittel, zur Verbesserung der Gleitfähigkeit oder Verzögerung der Freisetzung der Arzneistoffe offen bleibt.

Nachteil der genannten Produkte ist in jedem Falle der komplexe Aufbau mit entsprechenden Problemen bei Herstellung, Qualitätskontrolle und Kosten sowie zusätzlichen, Arzt und Patient belastenden Arbeitsschritten bei der Anwendung. Ein Teil der genannten Methoden führt zu einer über die beabsichtigte Gefäßerweiterung hinausgehenden gänzlich unerwünschten Gefäßverletzung. Andererseits hat jede zur Verlängerung der Kontaktzeit vorgesehene Maßnahme eine zusätzliche Minderversorgung der nachgeschalteten Gewebe mit Blut und Sauerstoff zur Folge.

Der Vollständigkeit halber wird noch auf eine in der WO 01/24866 beschriebenen Vorrichtung zum Verhindern der Restenose verwiesen, die mit einer von natürlichen Zellmembranen abgeleiteten lipiden Ceramide-Substanz beschichtet sind. Diese Substanz wird aufgrund ihrer bei gebräuchlichen Arzneistoffen nicht anzutreffenden Affinität zu den Zellwänden der Arterienwand verwendet. In der Fachliteratur wird jedoch weiterhin die Auffassung vertreten, dass eine Arzneimittelprophylaxe der Restenose eine Freisetzung der erforderlichen Wirkstoffe über Tage erfordert.

Der Erfindung liegt die Aufgabe zugrunde, einen Ballonkatheter für eine auf bestimmte Gewebebereiche oder Organteile begrenzte Arzneimittelabgabe bereitzustellen, die ohne schädigenden Einfluss auf gesundes Gewebe eine starke therapeutische Wirkung ausübt, den Patienten nur wenig belastet und mit geringem Aufwand angewendet und hergestellt werden kann.

Erfindungsgemäß wird die Aufgabe mit einer gemäß den Merkmalen der Ansprüche 1 und 14 ausgebildeten bzw. hergestellten Ballonkatheter gelöst. Aus den Unteransprüchen ergeben sich weitere Merkmale und vorteilhafte Weiterbildungen der Erfindung.

Durch die Erfindung werden in einem einfachen Herstellungsverfahren verbesserte arzneistofftragende Ballonkatheter bereitgestellt, die vielseitig anwendbar sind und eine sofortige Wirkstofffreigabe ermöglichen. Überraschend und entgegen der Lehrmeinung ist keine andauernde Wirkstofffreisetzung aus einer inerten Matrix (Polymer,Hydrogel, Mikrokapseln etc.) oder speziellen chemischen oder physikalischen Zuständen der Wirkstoffe erforderlich oder nützlich. Daher werden auch keine aufwendigen Techniken zur Produktion oder Kontrolle von Depotformulierungen benötigt.

Die Beschichtung von auf Kathetern befindlichen Ballonen mit Arzneistoffen gemäß der vorliegenden Erfindung ist insofern von besonderem Nutzen als nach dem Erweitern von Blutgefäßen oder anderen Hohlräumen im Körper mit den Ballonen häufig der Bedarf an therapeutischen Maßnahmen besteht, um eine Verengung oder einen Verschluss des mit dem Ballon unter Druck geschaffenen Lumens zu verhindern, Tumorwachstum zu begrenzen oder Heilungsprozesse einschließlich der Bildung von Kollateralkreisläufen zu fördern. Dies kann durch Arzneistoffe erreicht werden, die ihre Wirkung in unmittelbarer Nähe der Ballonoberfläche entfalten. Die Arzneistoffe haften auf dem Weg zum Ziel - meist durch intensiv durchblutete Arterien - bis zur Entfaltung des Ballons fest auf diesem, werden dann während der kurzen, oft nur Sekunden andauernden Kontaktzeit des entfalteten Ballons an das Gewebe in wirksamer Dosis abgegeben und von diesem in einer Weise aufgenommen, die das Abspülen durch den nach Deflation des Ballons sofort wieder einsetzenden Blutstrom vermeidet.

Zur Beschichtung sind erfindungsgemäß Ballone von Kathetern, die zumindest für kurze Zeit mit Druck gegen erkrankte Gewebe gepresst werden, vorgesehen. Bevorzugte Kathetermaterialien sind Polyamide, Polyamid-Gemische und Copolymere, Polyethylenterephthalat, Polyethylen und Copolymere, Polyurethan, Naturkautschuk und dessen Derivate. Die Länge und der Durchmesser der für die Pharmakotherapie vorgesehenen Bereiche der Katheter bzw. Ballone ist für die Anwendung nicht von entscheidender Bedeutung, da die Dosierung in µg Wirkstoff/ mm² Oberfläche berechnet wird. Beispielsweise sind für die Koronardilatationen Ballone im Bereich von 2 - 4 mm Durchmesser und von 1,0-4,0 cm Länge gebräuchlich. Für andere Gefäße können auch Ballone bis zu > 20 mm Durchmesser und Längen bis zu > 10 cm eingesetzt werden. Die zu beschichtenden Oberflächen können glatt (d.h. ohne besondere Struktur zur Aufnahme der Wirkstoffe), aufgeraut oder in beliebiger Weise mit Strukturen versehen sein, wobei spezielle Oberflächenstrukturen nicht Voraussetzung für die Haftung der Wirkstoffe sind, die Haftung aber auch nicht behindern. Die Haftung der Wirkstoffe auf den Ballonoberflächen wird ausschließlich durch die Wahl geeigneter Lösungsmittel und ggf. die Haftung beeinflussende Zusatzstoffe bewirkt. Sie ist selbst auf äußerlich vollständig glatten Ballonoberflächen überraschend fest.

Alle Oberflächen können zusätzlich mit Substanzen beschichtet worden sein oder werden, die die Gleitfähigkeit der Produkte verbessern, die Gerinnung von Blut an der Oberfläche verhindern oder sonstige Eigenschaften der Medizinprodukte verbessern, ohne dass die zur Beschichtung benutzten Materialien an die Umgebung abgegeben werden müssen und ohne dass die Beschichtung die Abgabe der Wirkstoffe zur Behandlung der Zielgewebe und damit die Wirksamkeit wesentlich einschränkt.

Ballonkatheter werden aus sehr dünnen Kunststoffschläuchen durch Aufweitung eine Segments von 1 bis ca. 10 cm Länge geformt. Die aufgeweitete, sehr dünnwandige Ballonmembran wird anschließend in mehrere längs zur Katheterachse angeordnete Falten gelegt und fest um die Katheterachse gewickelt, so dass der aufgeweitete Bereich im gefalteten Zustand einen nur minimal größeren Durchmesser aufweist als der übrige Katheter. Die enge Faltung der Ballonhülle ist Voraussetzung für die problemlose Passage des Ballonkatheters durch Einführungsschleusen, Führungskatheter und z.B. stark verengte Abschnitte von Blutgefäßen.

Die Ballone von Kathetern werden in gefaltetem Zustand beschichtet, wobei in jedem Fall eine intakte, ausreichend gleichmäßige Beschichtung der Oberfläche erzielt wird und die Wirkstoffe auch während des Einfaltens eines im entfalteten Zustand beschichteten Ballonkatheters ausreichend fest an dessen Oberfläche haften.

Die Herstellung eines in entfaltetem Zustand beschichteten Ballons erfolgt gemäß Stand der Technik ohne Beeinträchtigung der Beschichtung beispielsweise durch die Verwendung von Ballonhüllen mit vorgeformten Falten und Biegungen, deren Struktur im Material durch das Aufdehnen nicht verloren geht und die nach Ablassen des Druckes aus dem Ballon bewirken, dass sich die Ballonhülle wieder regelrecht wenigstens lose einfaltet, ohne dass es einer äußeren Kraft als primäre Ursache bedarf. Erst danach werden die vorgeformten Falten von außen oder durch Vakuum zusammengepresst. In keinem Falle sind Falten zum Festhalten des Wirkstoffs erforderlich. Weiterhin kann die Einfaltung durch geringe mechanische Kräfte mittels sehr glatter Materialien bewirkt werden, wobei die Werkzeuge auch beispielsweise mit schlüpfrigen biokompatiblen Flüssigkeiten benetzt sein können, in denen sich die Wirkstoffe nicht oder jedenfalls nicht gut lösen.

Gemäß einer weiteren Erfindungsvariante werden die Ballone fertig gefalteter Ballonkatheter durch Tauchen in niedrig viskose Wirkstofflösungen beschichtet. Dabei dringen Lösungsmittel und Wirkstoff zwischen die extrem engen Falten und bilden dort einen überraschend gleichmäßigen und in der Dosis reproduzierbaren Belag, der durch keinen weiteren Arbeitsschritt beschädigt wird. Die außen anhaftende Lösung bzw. der nach Trocknen des Lösungsmittels außen anhaftende Belag kann dort belassen oder in einem weiteren Arbeitsschritt entfernt werden, so dass nur der von den Falten des Ballons verdeckte Wirkstoff erhalten bleibt.

Nach der Beschichtung kann bei gefaltetem Ballon ein Stent auf den Ballonkatheter geschoben und auf diesem festgepresst werden. Danach ist nur noch die Sterilisation, z. B. mittels Ethylenoxid, erforderlich.

Der so gestaltete Arbeitsgang ist außerordentlich einfach, wenig störanfällig und auch mit mechanisch, chemisch und physikalisch empfindlichen Beschichtungsmaterialien durchzuführen. Es hat sich gezeigt, dass die Beschichtung nach diesem Verfahren zu keiner unerwünschten Lockerung oder Verklebung der Faltung führt und dass der derart aufgetragene Wirkstoff fest genug haftet, um auf dem Weg durch das Blut nicht abgetragen zu werden, andererseits bei Inflation des Ballons im Zielgewebe den Wirkstoff weitgehend freigibt.

Als Arzneistoffe kommen stark lipophile, weitgehend wasserunlösliche, an beliebige Gewebebestandteile bindende stark wirksame Arzneistoffe in Frage. Als lipophil werden Arzneistoffe bezeichnet, deren Verteilungskoeffizient Butanol: wässriger Puffer pH 7 = 0.5, bevorzugt = 1 und besonders bevorzugt = 5 bzw. Octanol: wässriger Puffer pH 7 = 1, bevorzugt = 10, besonders bevorzugt > 50 ist. Alternativ oder zusätzlich sollen die Arzneistoffe zu > 10 %, bevorzugt zu > 50 %, besonders bevorzugt zu > 80 % reversibel und/oder irreversibel an Zellbestandteile binden. Bevorzugt sind Substanzen zur Hemmung der Zellproliferation oder auch entzündlicher Prozesse oder Antioxidantien wie Paclitaxel und andere Taxane, Rapamycin und verwandte Substanzen, Tacrolimus und verwandte Substanzen, Corticoide, Sexualhormone (Östrogen, Estradiol, Antiandrogene) und verwandte Substanzen, Statine, Epothilone, Probucol, Prostacycline, Angiogeneseinduktoren etc.

Die Substanzen liegen bevorzugt als trockene Festsubstanz oder als Öl auf den Oberflächen der unterschiedlichen Medizinprodukte vor. Bevorzugt sind Partikel geringster Größe (in der Mehrzahl < 5 µm, bevorzugt < 1 µm, besonders bevorzugt < 0.1 µm), besonders bevorzugt sind amorphe, nicht kristalline Strukturen feinster Partikelgröße, die bei Kontakt mit Gewebe wegen ihrer großen Oberfläche trotz grundsätzlich geringer Wasserlöslichkeit der Arzneistoffe rasch in Lösung gehen und nicht als Mikrokapseln fungieren, d. h. sich spontan und schnell lösen. Dabei ist es ausreichend, dass eine wirksame Dosis in Form kleinster oder amorpher Partikel vorliegt; größere Partikel tragen zwar zur Wirkstoffkonzentration im Gewebe kaum bei, stören aber auch nicht. Die Dosierung richtet sich nach der erwünschten Wirkung und der Wirksamkeit des verwendeten Arzneistoffes. Sie kann bis zu 5 µg/ mm² erreichen, wobei dies keine Obergrenze darstellt. Geringere Dosierungen sind leichter zu realisieren.

Eine gute Haftung an den Oberflächen der Katheter, Nadeln oder Drähte bei Verbesserung der Aufnahme in die Gewebe wird durch Einbetten von stark lipophilen, schlecht wasserlöslichen Wirkstoffen in eine leicht wasserlösliche Matrixsubstanz erreicht. Als Matrixsubstanzen sind niedermoleklare (Molekulargewicht < 5000 D, bevorzugt < 2000 D) hydrophile Substanzen wie in vivo verwendete Kontrastmittel und Farbstoffe für unterschiedliche diagnostische Verfahren in der Medizin, Zucker und verwandte Substanzen wie Zuckeralkohole, niedermolekulare Polyethylenglycole, biokompatible organische und anorganische Salze wie z. B. Benzoate, Salze und andere Derivate der Salicylsäure etc. geeignet. Als Kontrastmittel sei auf die jodierten Röntgenkontrastmittel und die paramagnetischen Chelate verwiesen, Beispiele für Farbstoffe sind Indocyaningrün, Fluorescein und Methylenblau. Hilfsstoffe können auch zur Verbesserung der Lagerfähigkeit der Produkte dienen, spezielle ergänzende pharmakologische Wirkungen verursachen oder der Qualitätskontrolle dienen.

In einer weiteren Ausführung können die pharmazeutischen Wirkstoffe an Partikel adsorbiert oder mit niedermolekularer Matrix auf die Oberflächen geeigneter Medizinprodukte aufgebracht werden. Als Partikel sind wiederum als biokompatibel bekannte Diagnostika wie Ferrite und diverse Kontrastmittel für die Sonographie geeignet.

Hilfsstoffe aller Art können in geringerer oder höherer Dosis als die Wirkstoffe eingesetzt werden.

Die Beschichtung der Ballonkatheter erfolgt mittels Lösungen, Suspensionen oder Emulsionen der genannten Arzneistoffe und Hilfsstoffe. Geeignete Lösungs- Suspendier- oder Emulsionsmedien sind beispielsweise Ethanol, Isopropanol, Ethylacetat, Diethylether, Aceton, Dimethylsulfoxid, Dimethylformamid, Glycerin, Wasser oder Mischungen derselben. Die Auswahl der Lösungsmittel folgt entsprechend der Löslichkeit der Wirkstoffe und Zusatzstoffe sowie der Benetzung der zu beschichtenden Oberflächen und der Wirkung auf die Struktur der nach Verdampfen der Lösungsmittel zurückbleibenden Beschichtung und Partikel, deren Haftung auf der Oberfläche und die Wirkstoffübertragung in das Gewebe während sehr kurzer Kontaktzeiten.

Die Auftragung kann beispielsweise durch Tauchen, Bestreichen, Auftragen mittels Volumenmesseinrichtungen oder Besprühen jeweils bei unterschiedlichen Temperaturen und ggf. Dampfsättigungen der Lösungsmittel in der Atmosphäre geschehen. Der Vorgang kann mehrfach, ggf. auch unter Verwendung verschiedener Lösungsmittel und Hilfsstoffe, wiederholt werden.

Die Ballone fertig gefalteter Ballonkatheter lassen sich durch Tauchen in wirkstoffhaltige Lösungen oder andere Maßnahmen erstaunlich gleichmäßig, reproduzierbar, in der Dosis steuerbar und ohne Beeinträchtigung der Funktion der Katheter beschichten. Bei wiederholtem Tauchen in ungesättigten Wirkstofflösungen kommt es wider Erwarten nicht zu einer vollständigen Ablösung des vorher aufgetragenen Wirkstoffs sondern zu einer reproduzierbaren Erhöhung des Wirkstoffgehaltes der Ballone.

Überschüssige Lösung bzw. überschüssige, außen lose anhaftende Substanzen aus der Beschichtungslösung können mit einfachen Methoden entfernt werden, ohne dass es zu einer Beeinträchtigung der Wirksamkeit der Beschichtung kommt.

Die erfindungsgemäß ausgebildeten und hergestellten Ballonkatheter unterschiedlicher Art kommen kurzzeitig, d. h. für Sekunden, Minuten oder wenige Stunden mit dem Gewebe in Kontakt. In einigen Fällen ist es erwünscht, das Gewebe in unmittelbarer Nähe des Medizinproduktes pharmakologisch zu behandeln, zum Beispiel an überschießendem Wachstum als Reaktion auf eine Verletzung zu hindern oder Tumorwachstum zu reduzieren oder die Einsprossung von Blutgefäßen zu fördern oder Entzündungsreaktionen zu vermindern. In all diesen Fällen kann mit dem oben beschriebenen Verfahren eine hohe lokale Arzneistoffkonzentration für erstaunlich lange Zeit erzielt werden. Ein wesentlicher Vorteil ist die außerordentliche Vielfalt der Einsatzmöglichkeiten der beschriebenen Produkte und Verfahren.

Eine bevorzugte Anwendung ist die Verminderung der durch die Gefäßdilatation mittels Ballonkathetern induzierte Hyperproliferation der Gefäßwände. Diese ist im Bereich gegebenenfalls implantierter Gefäßstützen (Stents) auch durch Beschichtung der Stents mit Arzneistoffen zu erzielen, allerdings nur im unmittelbar durch den Stent bedeckten Gefäßbereich. Die beschichteten Ballonkatheter behandeln darüber hinaus die behandlungsbedürftigen Bereiche kurz vor und kurz hinter dem Stent, sie können ohne erneute Stentimplantation den Bereich innerhalb bereits vorhandener Stents behandeln oder Gefäße, in die kein Stent implantiert werden soll oder kann. Vorteilhaft gegenüber den über einen langen Zeitraum Arzneistoff freisetzenden Stents ist die bessere Einheilung bei gleichzeitig guter Hemmung der Hyperproliferation und das geringere Thromboserisiko.

Nachfolgend werden mehrere Ausführungsformen der Erfindung am Beispiel der Beschichtung von Ballonkathetern sowie in Bezug auf die Haftung der Beschichtung im Blut, die Restenosehemmung und den Wirkstoffgehalt der Katheter beschrieben.

### Beispiel 1:

### Beschichten eines expandierten Ballonkatheters mit Paclitaxel in Ethylacetat

Ballonkatheter der Fa. BMT, Oberpfaffenhofen/ München, Deutschland, mit der Bezeichnung Joker Lite, Ballongröße 2.5 mm x 20 mm, werden nach maximaler Expansion für 1 min. über die gesamte Ballonlänge in Ethylacetat, 18.8 mg Paclitaxel/ ml, + 1% pharmazeutisches Olivenöl, getaucht, getrocknet:
Paclitaxelgehalt 39 µg (nach Extraktion mit Ethanol, HPLC).

### Beispiel 2:

### Beschichten eines gefalteten Ballonkatheters mit Paclitaxel in Ethylacetat

Ballonkatheter der Fa. BMT, Oberpfaffenhofen/ München, Deutschland, mit der Bezeichnung Joker Lite, Ballongröße 2.5 mm x 20 mm, werden im gefalteten Zustand für 1 min. über die gesamte Ballonlänge in Ethylacetat, 18.8 mg Paclitaxel/ ml, + 1% pharmazeutisches Olivenöl, getaucht, getrocknet:
Paclitaxelgehalt 69 µg.

### Beispiel 3:

### Beschichten eines gefalteten Ballonkatheters mit Paclitaxel in Ethylacetat

a) Ballonkatheter der Fa. BMT, Oberpfaffenhofen/ München, Deutschland mit der Bezeichnung Joker Lite, Ballongröße 2.5 mm x 20 mm, werden im gefalteten Zustand für 1 min. über die gesamte Ballonlänge in Ethylacetat, 16.6 mg Paclitaxel/ ml, getaucht, 4 h getrocknet: Paclitaxelgehalt 54 µg
b) Ebenso, jedoch noch 2 mal 5 sec. mit 1 h Trocknungszeit nach jedem Tauchvorgang in Lösung A (= 3.33 ml Ethylacetat + 100.0 mg Paclitaxel) getaucht: Paclitaxelgehalt 126 µg
c) Ebenso, jedoch noch 4 mal 5 sec. mit 1 h Trocknungszeit nach jedem Tauchvorgang in die gleiche Lösung getaucht: Paclitaxelgehalt 158 µg

### Beispiel 4:

### Beschichten eines Ballonkatheters mit Paclitaxel in Aceton

350 mg Paclitaxel in 9.0 ml Aceton lösen; Ballonkatheter der Fa. BMT, Oberpfaffenhofen/ München, Deutschland mit der Bezeichnung Joker Lite, Ballongröße 2.5 mm x 20 mm, werden im maximal expandierten Zustand für 1 min. über die gesamte Ballonlänge getaucht, entnommen, das Lösungsmittel wird bei Raumtemperatur 12 h getrocknet. Danach wird der Ballon deflatiert und mit PTFE-beschichtetem Werkzeug in üblicher Weise eingefaltet. Optional kann ein Stent geeigneter Größe auf den Ballon gecrimmt werden: 29 µg Paclitaxel auf dem Ballon.

### Beispiel 5:

### Beschichten eines Ballonkatheters mit Paclitaxel in Aceton

a) Tauchen gefaltete Ballonkatheter der Fa. BMT mit der Bezeichnung Allegro, Ballongröße 2.5 x 20 mm in ein Gemisch von 0.15 ml Ethanol + 4.5 µl Ultravist 300 ( Röntgenkontrastmittel der Schering AG, Berlin, Deutschland) + 1.35 ml Aceton + 0.8 mg Sudanrot + 30.0 mg Paclitaxel:
   Die gefalteten Ballonabschnitte der Katheter werden 5 x getaucht, das 1. Mal für 1 Minute, dann 3 h Trocknungszeit, danach 4 mal für je 5 sec im Abstand von 1 h; danach wurde ein Stent aufgecrimpt und der Katheter mit Stent in üblicher Weise mit Ethylenoxid sterilisiert: Paclitaxelgehalt 172 µg, keine mittels HPLC detektierbaren Zersetzungsprodukte des Wirkstoffs
b) Statt Ultravist 300 wird eine gesättigte wässrige Mannit-Lösung zugesetzt
c) Statt Ultravist 300 wird eine gesättigte wässrige Natrium-Salicylat Lösung, pH 7.5, zugesetzt.
d) Der fertigen Lösung nach (5a) werden 5 mg Acetylsalicylsäure zugesetzt.
e) Der fertigen Lösung nach (5a) werden 5 mg Glycerin zugesetzt

### Beispiel 6:

### Haftung des Wirkstoffs in Blut

Es wurden 12 Ballonkatheter der Fa. BMT mit der Bezeichnung Allegro, Ballongröße 2.5 x 20 mm, verwendet. Je 6 Stück der gefalteten Ballonabschnitte der Katheter wurden entweder in [0.15 ml Ethanol + 4.5 µl Ultravist 300 + 1.35 ml Aceton + 0.8 mg Sudanrot+ 30.0 mg Paclitaxel] oder in [1.5 ml Ethylacetat + 0.8 mg Sudanrot + 31.0 mg Paclitaxel] 5 x getaucht, das 1. Mal für 1 Minute, dann 3 h Trocknungszeit, danach 4 mal für je 5 sec im Abstand von 1 h; danach wurden je 3 gefaltete Ballone jeder Serie in 50 ml Humanblut 5 min bei 37 °C leicht bewegt und dann zur Analyse des Paclitaxel-Gehaltes entnommen: Verminderung der Mittelwerte (n=3 je Beschichtungsmethode) durch 5 Minuten Bewegung in Blut im Vergleich zu je 3 Kontrollkathetern, die nicht in Blut inkubiert wurden.

| | |
|---|---|
| Aceton: | 12 % |
| Ethylacetat: | 10 % |

### Beispiel 7:

### Untersuchung der Restenosehemmung nach Angioplastie und Stentimplantation an den Koronarien von Schweinen

Gefaltete Ballonkatheter der Fa. BMT Typ Joker Lite, BMT 3.5 x 20 mm oder 3.0 x 20 mm wurden entweder in
Lösung A) 3.33 ml Ethylacetat (EA)+ 100.0 mg Paclitaxel oder in
Lösung B) 0.45 ml Ethanol + 100 µl Ultravist-370 + 4.5 ml Aceton (Ac) + 150.0 mg Paclitaxel
für 1 min eingetaucht und bei Raumtemperatur über Nacht getrocknet. Ein weiterer (niedrige Dosis = L) bzw. 4 weitere (hohe Dosis = H) Tauchvorgänge wurden jeweils nur für 5 Sekunden am nächsten Tag im Abstand von 1 h durchgeführt.

Wirkstoffgehalt nach 2 maligem Tauchen in Lösung (B) im Mittel 250 µg, bei 5 maligem Tauchen in Lösung (B) 500µg, in Lösung (A) 400 µg.

Insgesamt 22 Schweinen wurde mittels der mit Paclitaxel beschichteten Katheter oder mittels unbeschichteter Katheter Stents in die linke Vorderwand- oder Seitenwand-Koronararterie implantiert, wobei die Gefäße zur Stimulation der Restenose durch Gewebehyperplasie leicht überdehnt wurden. Nach 5 Wochen wurden die Tiere reangiographiert und das Maß der Gefäßverengung auf den Angiogrammen mit einem automatischen Computerprogramm gemessen.

| Gruppe | Stenose (%) |
|---|---|
| Unbeschichtet | 50.49 |
| AcL | 20.22 |
| EAH | 36.01 |
| AcH | 0.86 |
| p | .004 |

Quantitative Koronarangiographie 5 Wochen nach Stentimplantation mit unbeschichteten und beschichteten Kathetern; Stenose = prozentuale Verminderung des Lumendurchmessers im Bereich des Stents im Vergleich zu dem Lumendurchmesser unmittelbar nach Stentimplantation Mittelwert und statistische Signifikanz des Behandlungseffekts.

### Beispiel 8:

### Wirkstoffgehalt der Katheter nach Gefäßdilatation und Stentimplantation

Die Ballone aus Beispiel 8 wurden nach Stentimplantation und Entnahme aus den Tieren auf ca. 3 cm Länge von den Ballonkathetern abgetrennt und in 1.5 ml Ethanol überführt. Der Paclitaxelgehalt wurde mittels HPLC bestimmt. Alle verfügbaren beschichteten Ballone und eine Auswahl unbeschichteter Ballone wurden untersucht.

### Koronar,

| | | |
|---|---|---|
| 3.0 x 20 mm, Beschichtung: | Ac hoch | 38 ± 4 µg (n=4) |
| | Ac niedrig | 22 ± 5 µg (n=2) |
| | EEE hoch | 41 (n=1) |
| 3.5 x 20 mm, Beschichtung: | Ac hoch | 37 ± 10 µg (n=8) |
| | Ac niedrig | 26 ± 6 µg (n=8) |
| | EEE hoch | 53 ± 9 µg (n=9) |
| Unbeschichtet (unabhängig von Größe und Gefäßgebiet) | | |
| | | 0.9 ± 1.0 µg (n=7) |

Aus Beispiel 6 ergibt sich, dass maximal 10 % der Dosis verloren gehen bevor der Ballon expandiert wird und ca. 10 % der Dosis auf dem Ballon verbleiben.

### Beispiel 9:

Probucol wird in einer Konzentration von 100 mg/ ml in Aceton eingebracht; die Lösung wird wie in den vorhergehenden Beispielen beschrieben zum Beschichten der Ballonkatheter eingesetzt.

### Beispiel 10:

Rapamycin wird in einer Konzentration von 10 mg/ ml in Diethylether gelöst. Die Beschichtung der Ballonanteile der Katheter erfolgt wie in den vorhergehenden Beispielen beschrieben; die Ballone sollen nach der Entnahme aus der Beschichtungslösung möglichst sofort horizontal ausgerichtet und ständig um ihre Längsachse gedreht werden.

### Beispiel 11:

Epothilon B wird in einer Konzentration von 2 mg/ ml in Ethylacetat gelöst; die Lösung wird wie in den vorhergehenden Beispielen beschrieben zum Beschichten der Ballonkatheter eingesetzt.

## Patentansprüche

1. Ballonkatheter, **dadurch gekennzeichnet, dass** an der Oberfläche des Ballons lipophile, weitgehend wasserunlösliche, an beliebige Gewebebestandteile bindende Arzneistoffe mit nach Gewebekontakt sofortiger Wirkstofffreigabe haften, wobei der von den Falten abgedeckte Bereich mit dem nach dem Auftragen getrockneten Arzneistoff bedeckt ist.

2. Ballonkatheter nach Anspruch 1, **dadurch gekennzeichnet, dass** er ohne oder in Verbindung mit Stents, Katheter und/oder Teile von diesen, Nadeln und Führungsdrähte sowie Stents vorgesehen ist.

3. Ballonkatheter nach Anspruch 1 oder 2, **dadurch gekennzeichnet, dass** er einen in fertig gefaltetem Zustand durch Tauchen, Streichen, Sprühen oder Aufbringen mittels Volumenmesseinrichtung in einer niedrig viskosen Wirkstofflösung beschichteten Ballon enthält.

4. Ballonkatheter nach Anspruch 1 oder 2, **dadurch gekennzeichnet, dass** die lipophilen Arzneistoffe Substanzen zur Hemmung der Zellproliferation oder entzündlicher Prozesse sind oder Antioxidatien sind.

5. Ballonkatheter nach Anspruch 4, **dadurch gekennzeichnet, dass** die Arzneistoffe Paclitaxel und andere Taxane, Rapamycin und verwandte Substanzen, Tacrolimus und verwandte Substanzen, Corticoide, Sexualhormone und verwandte Substanzen, Statine, Epothilone, Probucol, Prostacycline oder Angiogeneseinduktoren sind.

6. Ballonkatheter nach Anspruch 4 oder 5, **dadurch gekennzeichnet, dass** die lipophilen Arzneistoffe als trockene Festsubstanz oder Öl auf der Oberfläche des Ballons vorliegen.

7. Ballonkatheter nach Anspruch 6, **dadurch gekennzeichnet, dass** die wirksame Dosis der Arzneistoffe amorphe Strukturen in einer Partikelgröße zwischen < 0,1 µm und 5 µm umfassen, die wegen ihrer großen Oberfläche trotz geringer Wasserlöslichkeit der Wirkstoffe schnell in Lösung gehen.

8. Ballonkatheter nach Anspruch 1, **dadurch gekennzeichnet, dass** die lipophilen Arzneistoffe zur Erzielung einer guten Haftwirkung an der Oberfläche des Ballons und zur Verbesserung der Aufnahme in das Gewebe in eine leicht wasserlösliche Matrixsubstanz eingebettet sind.

9. Ballonkatheter nach Anspruch 8, **dadurch gekennzeichnet, dass** die Matrixsubstanz aus einem niedermolekularen hydrophilen Stoff mit einem Molekulargewicht < 5000 D besteht.

10. Ballonkatheter nach Anspruch 8 oder 9, **dadurch gekennzeichnet, dass** die Matrixsubstanz ein Kontrastmittel, insbesondere ein jodiertes Röntgenkonstrastmittel, ist.

11. Ballonkatheter nach Anspruch 10 **dadurch gekennzeichnet, dass** der Wirkstoff Paclitaxel und das Röntgenkonstrastmittel Ultravist ist.

12. Ballonkatheter nach Anspruch 1, **dadurch gekennzeichnet, dass** die lipophilen Arzneistoffe an Partikel absorbiert oder mit niedermolekularer Matrix auf die Oberfläche der Vorrichtung aufgebracht sind.

13. Ballonkatheter nach Anspruch 1, **dadurch gekennzeichnet, dass** die Oberflächen zusätzlich mit Substanzen zur Beeinflussung der Gleitfähigkeit der Vorrichtung oder der Verringerung der Blutgerinnung beschichtet sind.

14. Verfahren zur Herstellung eines beschichteten Ballonkatheters nach Anspruch 1 bis 13, **dadurch gekennzeichnet, dass** die lipophilen Arzneistoffe und die Hilfsstoffe in einem Lösungs-, Suspendier- oder Emulsionsmedien durch Tauchen, Streichen, Sprühen oder mittels Volumenmesseinrichtung auf die Oberfläche eines gefalteten Ballons aufgebracht, wobei überschüssige Medien und lose an der Oberfläche haftende Substanzen entfernt werden.

15. Verfahren nach Anspruch 14, **dadurch gekennzeichnet, dass** der Beschichtungsvorgang bei reproduzierbarer Erhöhung des Wirkstoffgehalts mit gleichen oder verschiedenen Lösungs-, Suspendier- und Emulsionsmedien und/oder Hilfsstoffen mehrfach durchgeführt wird.

16. Verfahren nach Anspruch 14, **dadurch gekennzeichnet, dass** als Lösungs-, Suspendier- und Emulsionsmedien Ethanol, Isopropanol, Ethylacetat, Diethylether, Aceton, Dimethylsulfoxid, Dimethylformamid, Glycerin, Wasser oder Mischungen derselben eingesetzt werden.

17. Verfahren nach einem der Ansprüche 14 bis 16, **dadurch gekennzeichnet, dass** als Wirkstoffträger weitgehend anwendungsbereite gefaltete Ballone vorgesehen sind, die vor oder nach der Sterilisation ohne oder mit aufgebrachtem Stent beschichtet werden.

18. Verfahren nach Anspruch 14, **dadurch gekennzeichnet, dass** mit einem Ballonkatheter verbundene Stents vor oder nach einem Beschichtungsvorgang montiert werden.

19. Verfahren nach Anspruch 14, **dadurch gekennzeichnet, dass** die fertig beschichteten Ballonkatheter mittels Ethylenoxid sterilisiert werden.

20. Verwendung der gemäß den Ansprüchen 1 bis 19 ausgebildeten und hergestellten beschichteten Ballonkatheter zur Herstellung eines Mittels für die Behandlung von Gefäßerkrankungen oder Durchblutungsstörungen.

21. Verwendung der gemäß den Ansprüchen 1 bis 19 ausgebildeten und hergestellten beschichteten Ballonkatheter zur Herstellung eines Mittels für die Schaffung von offenen Passagen im Körper.

22. Verwendung der gemäß den Ansprüchen 1-19 ausgebildeten und hergestellten beschichteten Ballonkatheter zur Herstellung eines Mittels zur Tumorbehandlung.

## Claims

1. A balloon catheter, **characterised in that** lipophilic, largely water-insoluble medicinal substances that bind to any tissue components adhere to the surface of the balloon and immediately release the active ingredient after coming into contact with tissue, wherein the area covered by folds is covered with the medicinal substance dried after application.

2. A balloon catheter according to claim 1, **characterised in that** it is provided without or in conjunction with stents, catheters and/or parts thereof, needles and guiding wires as well as stents.

3. A balloon catheter according to claim 1 or 2, **characterised in that** it contains a balloon in a ready-folded state coated by immersion, brushing, spraying or application by means of a volume measuring device in a low-viscosity active ingredient solution.

4. A balloon catheter according to claim 1 or 2, **characterised in that** the lipophilic medicinal substances are substances for inhibiting cell proliferation or inflammatory processes, or are antioxidants.

5. A balloon catheter according to claim 4, **characterised in that** the medicinal substances are Paclitaxel and other taxanes, rapamycin and related substances, tacrolimus and related substances, corticoids, sexual hormones and related substances, statins, epothilones, probucol, prostacyclins or angiogenesis inducers.

6. A balloon catheter according to claim 4 or 5, **characterised in that** the lipophilic medicinal substances are present as a dry solid substance or oil on the surface of the balloon.

7. A balloon catheter according to claim 6, **characterised in that** the effective dose of the medicinal substances comprises amorphous structures with a particle size of between <0.1 µm and 5 µm, which because of their large surface area dissolve rapidly despite the poor water-solubility of the active ingredients.

8. A balloon catheter according to claim 1, **characterised in that** the lipophilic medicinal substances are embedded in a readily water-soluble matrix substance in order to achieve good adhesion to the surface of the balloon and to improve absorption into the tissue.

9. A balloon catheter according to claim 8, **characterised in that** the matrix substance consists of a low-molecular hydrophilic substance with a molecular weight <5000 D.

10. A balloon catheter according to claim 8 or 9, **characterised in that** the matrix substance is a contrast medium, in particular an iodised X-ray contrast medium.

11. A balloon catheter according to claim 10, **characterised in that** the active ingredient is Paclitaxel and the X-ray contrast medium is ultravist.

12. A balloon catheter according to claim 1, **characterised in that** the lipophilic medicinal substances are absorbed to particles or are applied on to the surface of the device with a low molecular matrix.

13. A balloon catheter according to claim 1, **characterised in that** the surfaces are additionally coated with substances that influence the sliding quality of the device or reduce blood coagulation.

14. A method of producing a coated balloon catheter according to claims 1 to 13, **characterised in that** the lipophilic medicinal substances and adjuvants are applied in a solution, suspension or emulsion medium by immersion, brushing or spraying or by means of a volume measuring device on to the surface of a folded balloon, wherein excess media and substances adhering loosely to the surface are removed.

15. A method according to claim 14, **characterised in that** the coating process is carried out repeatedly for a reproducible increase of the active ingredient content with the same or different solution, suspension or emulsion media and/or adjuvants.

16. A method according to claim 14, **characterised in that** ethanol, isopropanol, ethyl acetate, diethyl ether, acetone, dimethyl sulphoxide, dimethyl formamide, glycerin, water or mixtures thereof are used as solution, suspension and emulsion media.

17. A method according to any one of claims 14 to 16, **characterised in that** folded balloons substantially ready for application are provided as active ingredient carrier, which are coated before or after sterilisation with or without an applied stent.

18. A method according to claim 14, **characterised in that** stents connected with a balloon catheter are attached before or after the coating process.

19. A method according to claim 14, **characterised in that** the ready-coated balloon catheter is sterilised using ethylene oxide.

20. Use of the balloon catheters formed and produced according to claims 1 to 19 to prepare a means for the treatment of vascular diseases or circulatory disturbances.

21. Use of the balloon catheter formed and produced according to claims 1 to 19 to provide a means for creating open passages in the body.

22. Use of the balloon catheter formed and produced according to claims 1 to 19 to provide a means for the treatment of tumours.

## Revendications

1. Cathéter à ballonnet **caractérisé en ce que** des médicaments lipophiles, largement insolubles dans l'eau, liant à des constituants tissulaires quelconques avec une libération immédiate de substance au contact du tissu adhèrent à la surface du ballonnet, sachant que le secteur couvert par les plis est couvert du médicament séché après l'application.

2. Cathéter à ballonnet selon la revendication 1, **caractérisé en ce qu'**il est prévu sans ou en combinaison avec des endoprothèses, des cathéters et/ou des éléments de ceux-ci, des aiguilles et des fils de guidage ainsi que des endoprothèses.

3. Cathéter à ballonnet selon la revendication 1 ou 2, **caractérisé en ce qu'**il contient un ballonnet, qui a été enduit dans un état complètement plié par immersion, enduction, pulvérisation ou application au moyen d'un instrument de mesure de volume dans une solution de principes actifs faiblement visqueuse.

4. Cathéter à ballonnet selon la revendication 1 ou 2, **caractérisé en ce que** les médicaments lipophiles sont des substances servant à inhiber la prolifération cellulaire ou des processus inflammatoires ou des antioxydants.

5. Cathéter à ballonnet selon la revendication 4, **caractérisé en ce que** les médicaments sont du paclitaxel et autres taxanes, du rapamycine et substances analogues, du tacrolimus et substances analogues, des corticoïdes, des hormones sexuelles et substances analogues, de la statine, de l'épothilone, du probucol, de la prostacycline ou des inducteurs angiogéniques.

6. Cathéter à ballonnet selon la revendication 4 ou 5, **caractérisé en ce que** les médicaments lipophiles sont disponibles sous forme de substance solide sèche ou d'huile sur la surface du ballonnet.

7. Cathéter à ballonnet selon la revendication 6, **caractérisé en ce que** la dose efficace de médicaments comprend des structures amorphes ayant une taille de particule comprise entre < 0,1 µm et 5 µm qui malgré une faible solubilité dans l'eau sont dissoutes rapidement à cause de leur grande surface.

8. Cathéter à ballonnet selon la revendication 1, **caractérisé en ce que** les médicaments lipophiles sont enrobés dans une substance matricielle facilement soluble dans l'eau pour obtenir une bonne adhérence à la surface du ballonnet et améliorer l'absorption dans le tissu.

9. Cathéter à ballonnet selon la revendication 8, **caractérisé en ce que** la substance matricielle consiste en une substance hydrophile de faible poids moléculaire avec un poids moléculaire < 5000 D.

10. Cathéter à ballonnet selon la revendication 8 ou 9, **caractérisé en ce que** la substance matricielle est un produit de contraste, en particulier un produit de contraste à rayons X iodé.

11. Cathéter à ballonnet selon la revendication 10 **caractérisé en ce que** l'agent actif est du paclitaxel et le produit de contraste à rayons X est Ultravist.

12. Cathéter à ballonnet selon la revendication 1, **caractérisé en ce que** les médicaments lipophiles sont absorbés sur des particules ou appliqués avec une matrice à faible poids moléculaire sur la surface du dispositif.

13. Cathéter à ballonnet selon la revendication 1, **caractérisé en ce que** les surfaces sont également recouvertes de substances servant à influencer la capacité coulissante du dispositif ou à réduire la coagulation du sang.

14. Procédé de fabrication d'un cathéter à ballonnet enduit selon les revendications 1 à 13, **caractérisé en ce que** les médicaments lipophiles et les adjuvants sont appliqués dans des milieux de solution, de suspension ou d'émulsion sur la surface d'un ballonnet plié par immersion, enduction, pulvérisation ou au moyen d'un instrument de mesure de volume, sachant que les excès de milieux et les substances adhérant de façon lâche à la surface sont enlevés.

15. Procédé selon la revendication 14, **caractérisé en ce que** le processus d'enduction est exécuté plusieurs fois à une augmentation reproductible de la teneur en principes actifs avec des milieux de solution, de suspension et d'émulsion et/ou des adjuvants identiques ou différents.

16. Procédé selon la revendication 14, **caractérisé en ce que** l'éthanol, l'isopropanol, l'acétate d'éthyle, l'éther diéthylique, l'acétone, le diméthylsulphoxide, le diméthylformamide, la glycérine, l'eau ou des mélanges de ceux-ci sont utilisés comme milieux de solution, de suspension et d'émulsion.

17. Procédé selon l'une des revendications 14 à 16, **caractérisé en ce que** des ballonnets pliés largement prêts à l'emploi sont prévus comme des supports de principes actifs, ces ballonnets étant enduits avant ou après la stérilisation avec ou sans montage de l'endoprothèse.

18. Procédé selon la revendication 14, **caractérisé en ce que** des endoprothèses reliées à un cathéter à ballonnet sont montées avant ou après un processus d'enduction.

19. Procédé selon la revendication 14, **caractérisé en ce que** les cathéters à ballonnet complètement enduits sont stérilisés au moyen d'oxyde d'éthylène.

20. Utilisation de cathéters à ballonnet enduits conçus et fabriqués selon les revendications 1 à 19 pour la fabrication d'un moyen de traitement des maladies vasculaires ou des troubles de la circulation sanguine.

21. Utilisation de cathéters à ballonnet enduits conçus et fabriqués selon les revendications 1 à 19 pour la fabrication d'un moyen de création de passages ouverts dans le corps.

22. Utilisation de cathéters à ballonnet enduits conçus et fabriqués selon les revendications 1 à 19 pour la fabrication d'un moyen de traitement des tumeurs.
